# EUROPEAN PATENT APPLICATION

(11) **EP 1 935 438 A1**
(43) Date of publication of application: **25.06.2008**
(21) Application number: 06797938.5
(22) Date of filing: 13.09.2006
(51) Int. Cl.: A61L 27/00

(54) **BIOMATERIAL FOR REGENERATIVE MEDICINE**

(30) Priority: 16.09.2005 JP 2005271095
(71) Applicant: St. Marianna University School of Medicine, Kawasaki-shi, Kanagawa 216-8511 (JP)
(72) Inventor: YUDOH, Kazuo, Yokohama-shi Kanagawa 225-0002 (JP)
(74) Representative: Bassil, Nicholas Charles
(86) International application number: PCT/JP2006/318188
(87) International publication number: WO 2007/032404

(57) **Abstract**

It was examined whether a cartilage-like tissue is formed under various reaction conditions using cartilage matrix components: glycosaminoglycan, proteoglycan, and collagen. The present inventors have discovered that proteoglycan bound to glycosaminoglycan through self-organization form an aggregate when the glycosaminoglycan was reacted with proteoglycan under specific concentrations and pH, and that a mesh structure composed of collagen fibers was constructed through self-organization using the aggregates as a skeleton when the aggregates were reacted with collagen molecules.

## Description

### Technical Field

The present invention relates to usable biomaterials for tissue regeneration, particularly to glycosaminoglycan/proteoglycan/collagen complexes formed through self-organization techniques.

### Background Art

With the arrival of an aging society, there is an increasing trend of patients with bone and joint diseases/motor organ diseases such as osteoporosis and osteoarthritis. In fact, the number of osteoarthritis patients in Japan is estimated to be seven to ten millions. Since bone and joint diseases/motor organ diseases pose challenges in daily life, development of countermeasures and preventive methods has called for urgent attention in the society. Most of the current treatments for joint diseases/motor organ diseases are training for improving daily movements (muscle exercises, use of supports/braces, and the like) and symptomatic therapies using antiphlogistic analgesic agents. For patients, the efficacy of these symptomatic treatments is unsatisfactory. Articular symptoms often worsen with age, and surgical treatments (use of artificial joints and the like) are currently selected for cases with osteoarticular damage or alignment irregularities. However, surgical treatments have many issues such as cost and risk of infection, and furthermore, some patients are forced to replace their artificial joints several years to a decade later.

Preventive methods and early countermeasures are required for motor organ diseases because after disease onset, tissues become damaged with age, and articular cartilage tissues have extremely poor repairability. However, neither effective treatments nor medical techniques have been established yet. Therefore, the establishment of novel pharmaceutical agents and therapeutic strategies showing clinical efficacy towards age-related motor organ diseases is urgently needed to maintain high activities of daily living (ADL) in this aging society.

Recently, joint reconstruction using new technologies such as regenerative medicine (tissue engineering) is drawing attention as a treatment for severe bone and joint damage/degeneration. Mimics of bone tissue matrices (artificial bones) comprising hydroxyapatite as a main ingredient have been developed for bone defect/damage. High bone affinity and bone-like rigidity are reproduced in such artificial bones. Artificial bones are already clinically applied to diseases/cases having large bone defects (75 to 100 cm³), and satisfactory treatment outcomes have been reported. Since bones have high repairability (remodelling property) themselves, artificial bones are thought to be replaced with bones through self-organization in several weeks to several months.

On the other hand, cartilage regeneration techniques have not completely reproduced cartilage-specific tissue properties (elastic deformation effect and elastohydrodynamic lubrication mechanism) in artificial cartilages. Cartilage tissue consists of chondrocytes and cartilage matrix. Chondrocytes are highly differentiated cells; they are in a steady state and hardly proliferate through cell division in cartilage tissues. Although chondrocytes account for about merely 10% of cartilage tissue, they produce cartilage matrix components in cartilage tissue to maintain cartilage matrix which accounts for about 90% of cartilage tissue. At present, attempts are being made to artificially reproduce cartilage tissue using chondrocytes for use in the treatment of cartilage damage/degeneration. However, with the current technology, formation of cartilage-like tissues requires a process of making chondrocytes produce cartilage matrix components themselves. For example, a three-dimensional culture material produced by culturing chondrocytes *ex vivo* using a collagen gel or an agarose gel is transplanted into cartilage defective sites in a subset of selected subjects (young subjects with small defects of less than 3 cm³ as a result of external injury). In addition, attempts have also been reported to induce differentiation of bone marrow-derived mesenchymal stem cells into chondrocytes in an *in vitro* experimental system and use them as a cell source of cartilage regeneration.

Such techniques of forming cartilage-like tissues using chondrocytes are still under development in terms of practical application. When using the current technology to fill a cartilage defect by the abovementioned techniques of cartilage-like tissue formation, a much greater ratio of chondrocytes than that actually existing in a living cartilage tissue is required to form a sufficient amount of cartilage matrix for filling the defective site. Specifically, with the current technology, 2 x 10⁶ to 5 x 10⁶ chondrocytes are required to fill 1 cm³ of a cartilage defect with cartilage-like tissue. To obtain such a large amount of cell source (number of chondrocytes) that is necessary and sufficient for the treatment, cells have to be passaged several times. However, when chondrocytes are plate-cultured like other types of cells, they lose chondrocyte-specific properties (decrease in cartilage matrix productivity and alteration in cell morphology) during passage culture, and may possibly dedifferentiate even though they show proliferation potency. As such, when a large number of chondrocytes are required, the plate culture method has a lot of problems to solve in terms of cell source. It requires as long as several weeks and the risk of dedifferentiation is unavoidable. The three-dimensional culture method could provide a solution to the above issue of dedifferentiation, but similarly to the plate culture method, it require a large number of cells and a long period of time. For example, it takes several weeks to produce a three-dimensionally cultured (gel-like) tissue of chondrocytes *ex vivo* using a collagen gel or the like. The transplantation of such three-dimensionally cultured (gel-like) tissue of chondrocytes would not provide the remaining cartilage tissue in the joint with repairability; thus, in the current situation, it takes as long as several months (up to six months) until an artificial cartilage-like tissue is engrafted in the defective site and forms tissue (Non-patent Documents 1 and 2). Besides the above time-related issue, the three-dimensional culture method is labour consuming. When chondrocytes are three-dimensionally cultured on a collagen gel and the gel is directly transplanted, it easily leaks out from the transplanted site due to its fluidity; thus, the gel needs to be covered with a Teflon (registered trademark) film, periosteum, or the like to prevent leakage. Further, whether or not the three-dimensionally cultured (gel-like) tissue of chondrocytes can be maintained as a tissue capable of exerting cartilage functions (low frictionality and load resistance) is still under investigation.

There are also ongoing studies on the chemical preparation of tissue regeneration materials which mimic cartilage tissue. For example, there are reports on hyaluronic acid crosslinked with epichlorohydrin, and glycosaminoglycan-polycation complexes having glycosaminoglycans and polycations crosslinked through condensation reaction (Patent Document 1). However, the use of crosslinking agents and condensing agents requires washing to remove the crosslinking agents, condensing agents, and byproducts in the production process. Moreover, transplantation of such product into the body poses a risk of residual chemical substance. Further, since the complex prepared by using crosslinking agents and condensing agents cannot mimic a living tissue in the nano structure level, it is unclear as whether or not it can fulfil the required cartilaginous functions such as low frictionality, load resistance, and bioaffinity. In this regard, no conventional tissue regeneration materials have been found to have sufficiently reproduced the structure and functions of a cartilage tissue.
Patent Document 1: Japanese Patent Application Kokai Publication No. (JP-A) 2002-80501 (unexamined, published Japanese patent application)
Patent Document 2: JP-A (Kokai) 2002-248119
Non-patent Document 1: Ochi M., Uchio Y, Tobita M., and Kuriwaka M. Current concepts in tissue engineering technique for repair of cartilage defect. Artif. Organs. 25(3):172-9, 2001.
Non-patent Document 2: Ochi M., Uchio Y., Kawasaki K., Wakitani S., and Iwasa J. Transplantation of cartilage-like tissue made by tissue engineering in the treatment of cartilage defects of the knee. J Bone Joint Surg. Br. 84(4):571-8, 2002.
Non-patent Document 3: Kikuchi M., Itoh S., Ichinose S., Shinomiya K., and Tanaka J. Self-organization mechanism in a bone-like hydroxyapatite/collagen nanocomposite synthesized in vitro and its biological reaction in vivo. Biomaterials. 22(13):1705-11, 2001.

### Disclosure of the Invention

### [Problems to Be Solved by the Invention]

The present invention was achieved in view of the above circumstances. An objective of the present invention is to provide tissue regeneration biomaterials that are comparable to living tissues in terms of both structure and function.

### [Means for Solving the Problems]

To solve the above problems, the present inventors have devoted themselves to research. The present inventors were seeking for a technique to create a cartilage-like tissue, which unlike conventional techniques, neither makes chondrocytes produce cartilage matrix to induce cartilage tissue formation, nor uses crosslinking agents and condensing agents. An attempt has been made to form cartilage-like tissues through application of self-organization techniques. Self-organization techniques use a phenomenon that, depending on environmental conditions, randomly moving molecules in a steady state may form a regularly-organized structure according to physical or chemical properties such as intermolecular bonding strength, surface modification, and orientation and ionic arrangement of covalent bonds. It is known that hydroxyapatite, collagen, hyaluronic acid, and chondroitin sulfate form a body through self-organization (Non-patent Document 3 and Patent Document 2). However, hydroxyapatite is a bone component that is intrinsically nonexistent in cartilage tissue. The formation of a cartilage-like tissue through the application of self-organization techniques has never been reported. The present inventors have examined the formation of a cartilage-like tissue under various reaction conditions using cartilage matrix components: glycosaminoglycan, proteoglycan, and collagen. As a result, the present inventors have discovered that when glycosaminoglycan was reacted with proteoglycan at a specific concentration and pH, aggregates of proteoglycan and glycosaminoglycan were formed through self-organization. The present inventors further discovered that, when the aggregates were further reacted with collagen, collagen fibers constructed a mesh structure through self-organization, using the aggregates as a skeleton to form a complex with cartilage-like physical properties. Further, when chondrocytes were three-dimensionally cultured using the complex, the complex served as a scaffold for these chondrocytes, confirming that a three-dimensional environment suitable for long-term survival of chondrocytes could be reproduced. Accordingly, complexes formed by the above method have extremely suitable properties as biomaterials for cartilage tissue engineering. In other words, the present invention relates to self-organized glycosaminoglycan/proteoglycan/collagen complexes which are usable as biomaterials for tissue regeneration. Specifically, the following inventions are provided.
[1] A method for producing a self-organized glycosaminoglycan/proteoglycan/collagen complex, comprising steps (a) and (b) below:
   (a) a step of preparing a glycosaminoglycan-proteoglycan aggregate by mixing glycosaminoglycan with proteoglycan; and
   (b) a step of mixing collagen with said glycosaminoglycan-proteoglycan aggregate;
[2] a method for producing a cartilage-like complex, comprising steps (a) and (b) below:
   (a) a step of preparing a glycosaminoglycan-proteoglycan aggregate by mixing glycosaminoglycan with proteoglycan; and
   (b) a step of mixing collagen with said glycosaminoglycan-proteoglycan aggregate to produce a cartilage-like self-organized glycosaminoglycan/proteoglycan/collagen complex;
[3] a method for producing a cartilage matrix-like complex, comprising steps (a) and (b) below:
   (a) a step of preparing a glycosaminoglycan-proteoglycan aggregate by mixing glycosaminoglycan with proteoglycan; and
   (b) a step of mixing collagen with said glycosaminoglycan-proteoglycan aggregate to produce a cartilage matrix-like self-organized glycosaminoglycan/proteoglycan/collagen complex;
[4] the production method of any one of [1] to [3], wherein the glycosaminoglycan is hyaluronic acid;
[5] the production method of any one of [1] to [4], wherein the proteoglycan is aggrecan;
[6] the production method of any one of [1] to [5], wherein the collagen is type II collagen;
[7] the method of any one of [4] to [6], wherein the hyaluronic acid is a hyaluronic acid solution at pH 5 to pH 10 in step (a);
[8] the method of any one of [5] to [7], wherein the aggrecan is an aggrecan solution at pH 5 to pH 10 in step (a);
[9] the method of [1], wherein the collagen is a collagen solution at pH 5 to pH 10 in step (b);
[10] the method of any one of [4] to [6], wherein the hyaluronic acid is a hyaluronic acid solution at a concentration of 20 volume percent or less in step (a);
[11] the method of any one of [5] to [7], wherein the aggrecan is an aggrecan solution at a concentration of 0.1 to 1.0 mg/ml in step (a);
[12] the method of [1], wherein the collagen is a collagen solution at a concentration of 0.1 to 5.0 mg/ml in step (b);
[13] a self-organized glycosaminoglycan/proteoglycan/collagen complex produced by the method of any one of [1] and [4] to [12];
[14] a complex comprising hyaluronic acid, aggrecan, and type II collagen, which has a mesh structure formed by linkage between a type II collagen fiber and an aggregate of hyaluronic acid-bound aggrecan;
[15] a cartilage-like complex produced by the method of any one of [2] and [4] to [12];
[16] a cartilage matrix-like complex produced by the method of any one of [3] to [12];
[17] a material for cartilage tissue regeneration or treatment of cartilage damage or cartilage degeneration, comprising the complex of any one of [13] to [16];
[18] a method for producing a chondrocyte-comprising material for treatment of cartilage damage or cartilage degeneration, comprising steps (a) to (c) below:
   (a) a step of preparing a glycosaminoglycan-proteoglycan aggregate by mixing glycosaminoglycan with proteoglycan;
   (b) a step of mixing collagen with said glycosaminoglycan-proteoglycan aggregate to produce a self-organized glycosaminoglycan/proteoglycan/collagen complex; and
   (c) a step of culturing a chondrocyte using said self-organized glycosaminoglycan/proteoglycan/collagen complex;
[19] the production method of [18], wherein the chondrocyte is derived from a patient who receives treatment of cartilage damage or cartilage degeneration;
[20] a chondrocyte-comprising material for treatment of cartilage damage or cartilage degeneration produced by the method of [18] or [19];
[21] a three-dimensional cell culture method, comprising a step of preparing a complex according to the method of [1], and a step of culturing a cell using said complex;
[22] the three-dimensional culture method of [21], wherein the cell is a chondrocyte;
[23] a therapeutic method for a disease involving cartilage damage or cartilage degeneration, comprising a step of administering the material of [17] or [20] to a joint having cartilage damage or cartilage degeneration;
[24] use of the complex of [13] or [14] for the production of a material for treatment of cartilage damage or cartilage degeneration; and
[25] a three-dimensional cell culture material, comprising the complex of [13] or [14].

### Brief Description of the Drawings

Fig. 1 shows a schematic diagram of a living cartilage tissue.
Fig. 2 shows phase-contrast microscopic photographs explaining the production process of a self-organized glycosaminoglycan/proteoglycan/collagen complex using aggrecan, hyaluronic acid, and type II collagen. Aggrecan-hyaluronic acid aggregates (pH 9) (Fig. 2A) and a type II collagen solution (pH 9) (Fig. 2B) formed a self-organized glycosaminoglycan/proteoglycan/collagen complex (pH 9) (Fig. 2C).
Fig. 3 shows a transmission electron microscopic photograph of an aggrecan-hyaluronic acid aggregate. It is observed that aggrecan was bound to a hyaluronic acid chain at 10 to 20 nm intervals in high density to form an aggregate.
Fig. 4 shows photographs explaining the formation process of a self-organized hyaluronic acid/aggrecan/type II collagen complex. The photographs above indicate (from left to right) immediately, five minutes, and ten minutes after mixing a collagen solution with the AG-HA aggregates, respectively. The photographs below indicate (from left to right) 20 minutes, 30 minutes, and two to four hours after mixing, respectively. Aggregation of collagen molecules was observed in the "after five minutes" photograph. Formation of fibrous collagen was confirmed in the "after 30 minutes" photograph.
Fig. 5 shows a transmission electron microscopic photograph of a hyaluronic acid/aggrecan/type II collagen complex formed through self-organization. A mesh structure comprising fibrous collagen formed of covalently-bonded needle-shaped collagen molecules was observed.
Fig. 6 shows microscopic photographs of a self-organized hyaluronic acid/aggrecan/type II collagen complex and gel-like collagen.
Fig. 7 shows a transmission electron microscopic photograph of a hyaluronic acid/aggrecan/type II collagen complex formed through self-organization. It was observed that the aggrecan-hyaluronic acid aggregates were held in a mesh structure formed of type II collagen fibers.
Fig. 8A shows optical microscopic images of a self-organized hyaluronic acid/aggrecan/type II collagen complex and rat chondrocytes 24 hours after chondrocytes were cultured using the complex. Fig. 8B shows scanning electron microscopic (SEM) images of the complex and chondrocytes after one week of culturing. The arrows indicate chondrocytes.
Fig. 9 shows transmission electron microscopic (TEM) images of the complex and chondrocytes after one week of culturing. The chondrocytes are indicated by arrows. In the figure, (a) indicates wrinkles on the polyethylene film surface caused by poor attachment between the film and epoxy, and difference in their shrinkabilities.
Fig. 10 shows photographs showing the process of transplanting a chondrocyte-transferred self-organized hyaluronic acid/aggrecan/type II collagen complex into a rat knee cartilage tissue. In the figure, (a) indicates piercing a hole in the cartilage surface and transplanting the complex.

### Best Mode for Carrying Out the Invention

The present invention provides methods for producing self-organized glycosaminoglycan/proteoglycan/collagen complexes. The methods of the present invention are based on the present inventors' first success in forming complexes of hyaluronic acid, aggrecan, and collagen conjugated through self-organization. In the present invention, the self-organized glycosaminoglycan/proteoglycan/collagen complex (may be referred to as "complex of the present invention" hereinbelow) indicates a complex having a mesh structure formed by linking glycosaminoglycan, proteoglycan, and collagen through self-organization. The complexes of the present invention comprise glycosaminoglycan, proteoglycan, and collagen. The term "linkage" in the complexes of the present invention does not only mean linkage through chemical bondings between molecules, but also refers to linkage by physical entanglement between molecules, and conditions where molecules are physically held in a mesh structure. For example, a complex physically holding glycosaminoglycan and proteoglycan in a mesh structure of collagen fibers is formed by "linkage" of the present invention, and such a complex is included in the complexes of the present invention as long as it has a mesh structure formed through self-organization. The methods of the present invention comprise a step of preparing glycosaminoglycan-proteoglycan aggregates by mixing glycosaminoglycan and proteoglycan, and a step of mixing collagen with the glycosaminoglycan-proteoglycan aggregates.

The "step of preparing glycosaminoglycan-proteoglycan aggregates by mixing glycosaminoglycan and proteoglycan" in the present invention (may be referred to as "glycosaminoglycan-proteoglycan aggregate preparation step" hereinbelow) is described.

Glycosaminoglycans are acidic polysaccharides comprising repeating units of a disaccharide having an aminosugar bound to either uronic acid or galactose. The glycosaminoglycans are categorized according to their skeletal structure into chondroitin sulfate/dermatan sulfate, heparan sulfate/heparin, keratan sulfate, and hyaluronic acid. Any one of hyaluronic acid, chondroitin sulfate, keratan sulfate, heparin, and heparan sulfate may be used as a glycosaminoglycan in the methods of the present invention. When the methods of the present invention are conducted for production of complexes for cartilage regeneration, hyaluronic acid is preferably used as glycosaminoglycan.

In the glycosaminoglycan-proteoglycan aggregate preparation step of the present invention, the glycosaminoglycan is preferably prepared in a solution at a concentration of 20 volume percent or less, more preferably an aqueous solution at 0.5 to 10 volume percent, and yet more preferably an aqueous solution at 1 to 5 volume percent. The solvent for dissolving glycosaminoglycan is not limited to water, and may be any protein soluble solvents. A solvent to be used desirably contains no substance known to be toxic to living bodies. Examples of a suitably usable solvent include distilled water, phosphate buffer solution, and cell culture solution. When a hyaluronic acid solution is used as glycosaminoglycan, the pH is preferably 5 to 10, more preferably 6 to 9, and most preferably 8 to 9.

Proteoglycans generally and collectively refer to molecules of glycosaminoglycans covalently bound to proteins. In the methods of the present invention, there is no specific limitation on the usable proteoglycan. For example, aggrecan, biglycan, decorin, versican, neurocan, and brevican may be used. When a method of the present invention is conducted for production of a complex for cartilage regeneration, aggrecan is preferably used as proteoglycan.

The origin of the proteoglycan to be used for the present invention is not limited. Proteoglycan may be appropriately selected from those derived from various animals including mammals (such as humans, cattle, and pigs), birds (such as chickens), fishes (such as sharks and salmons), and crustaceans (such as crabs and shrimps) according to the application purpose of the complexes of the present invention. When a complex of the present invention is used for treating cartilage defect or deformation, the origin can be selected to suit the patient to be administered with the complex. For example, when a complex of the present invention is administered to a human patient, the proteoglycan is desirably selected from those derived from an origin having low immunogenicity to humans.

In the glycosaminoglycan-proteoglycan aggregate preparation step of the present invention, the proteoglycan is preferably prepared in a solution at a concentration of 0.1 to 1.0 mg/ml, more preferably an aqueous solution at 0.1 to 0.5 mg/ml, and yet more preferably an aqueous solution at 0.25 to 0.5 mg/ml. The solvent for dissolving proteoglycan is not limited to water, and may be any polysaccharide soluble solvents. A solvent to be used desirably contains no substance known to be toxic to living bodies. Examples of solvents that can be suitably used include distilled water, phosphate buffer solution, and cell culture solution. When an aggrecan solution is used, the pH is preferably 5 to 10, more preferably 6 to 9, and most preferably 8 to 9.

In the glycosaminoglycan-proteoglycan aggregate preparation step of the present invention, glycosaminoglycan and proteoglycan, preferably a glycosaminoglycan solution and a proteoglycan solution that have been prepared at a specific concentration and specific pH as described above are mixed by stirring at a fixed temperature. Preferably, they are mixed by simultaneous dripping. The temperature for mixing is preferably 25°C to 45°C, more preferably 35°C to 40°C, and most preferably 36°C to 38°C. In the glycosaminoglycan-proteoglycan aggregate preparation step, substances other than glycosaminoglycan and proteoglycan may be mixed as long as the "glycosaminoglycan-proteoglycan aggregates" to be described are formed.

By the above mixing, the "glycosaminoglycan-proteoglycan aggregates" are formed. The glycosaminoglycan-proteoglycan aggregates of the present invention are fibrous. Formation of the glycosaminoglycan-proteoglycan aggregates of the present invention can be readily determined by confirming the presence/absence of a fibrous substance using a microscope. Mixing or linking substances other than glycosaminoglycan or proteoglycan is not prohibited in the glycosaminoglycan-proteoglycan aggregates of the present invention, as long as the glycosaminoglycan and proteoglycan link to form fibrous substances. Specifically, even if the glycosaminoglycan and proteoglycan are mixed with or linked to substances contained in cartilage tissue, they are included in the glycosaminoglycan-proteoglycan aggregates of the present invention as long as they form fibrous substances.

In the methods of the present invention, the glycosaminoglycan-proteoglycan aggregates become the skeleton of a mesh structure of a self-organized glycosaminoglycan/proteoglycan/collagen complex. Therefore, formation of the glycosaminoglycan-proteoglycan aggregates is an important factor for the formation of a self-organized glycosaminoglycan/proteoglycan/collagen complex. In the Examples below, hyaluronic acid was used as glycosaminoglycan and aggrecan was used as proteoglycan to form the complexes of the present invention. In the "glycosaminoglycan-proteoglycan aggregate preparation step", an average of 200 or more aggrecans bind to a hyaluronic acid, forming a glycosaminoglycan-proteoglycan aggregate.

Next, the "step of mixing collagen with the glycosaminoglycan-proteoglycan aggregate" in the present invention (hereinbelow, referred to as "collagen molecule mixing step") is described. The collagen molecule mixing step in the methods of the present invention is a step where collagen is mixed with "glycosaminoglycan-proteoglycan aggregates" which have been prepared in the glycosaminoglycan-proteoglycan aggregate preparation step.

The collagen to be used for the present invention may be either type I collagen or type II collagen, although type II collagen is preferred. In the methods of the present invention, collagen is preferably prepared in a solution at a concentration of 0.1 to 5.0 mg/ml, more preferably an aqueous solution at 0.1 to 1.0 mg/ml, and yet more preferably an aqueous solution at 0.1 to 0.5 mg/ml. The pH of the collagen solution is preferably 5 to 10, more preferably 6 to 9, and most preferably 8 to 9. A solvent for dissolving collagen is not limited to water, and may be any collagen soluble solvents. A solvent to be used desirably contains no substance known to be toxic to living bodies.

The origin of the collagen to be used for the present invention is not limited. The collagen may be appropriately selected from those derived from various vertebrates including mammals (such as humans, cattle, and pigs) and fishes (such as sharks and salmons) according to the application purpose of the complexes of the present invention. If a complex of the present invention is used for the treatment of cartilage defect or deformation, the origin can be selected to suit the patient to be administered with the complex. For example, when a complex of the present invention is administered to a human patient, the collagen is desirably selected from those derived from an origin having low immunogenicity to humans, and human collagen is most preferable. The collagen to be used for the present invention may be produced by any methods. The collagen to be used may be a natural extract, or may be prepared by genetic modification techniques or chemical synthesis, as long as the purity and safety are confirmed for the purpose of application of the complex.

The collagen is mixed by stirring into the glycosaminoglycan-proteoglycan aggregates at a fixed temperature. Preferably, they are mixed by simultaneous dripping. They should be mixed at a temperature that does not cause protein denaturation, preferably 25°C to 45°C, more preferably 35°C to 40°C, and most preferably 36°C to 38°C. In the collagen molecule mixing step, substances other than glycosaminoglycan-proteoglycan aggregates and collagen may be mixed as long as the self-organized glycosaminoglycan/proteoglycan/collagen complexes are formed. For example, components of living tissues such as cartilage may be contained.

In the collagen molecule mixing step of the present invention, collagen fibroses, and then the fibrosing collagen and the glycosaminoglycan-proteoglycan aggregate form a complex of the present invention. As described above, collagen does not always have to be chemically bonded to proteoglycan in the self-organized glycosaminoglycan/proteoglycan/collagen complexes of the present invention. A structure, in which the glycosaminoglycan-proteoglycan aggregates are held in a mesh structure composed of collagen fibers formed through polymerization of collagen molecules, is comprised in the complexes of the present invention.

After the collagen molecule mixing step, the self-organized glycosaminoglycan/proteoglycan/collagen complex may be subjected to an operation such as centrifugation to reduce its moisture content. Lower moisture content enables the formation of a denser complex. For example, dehydration can be performed by centrifugation at 3,000 rpm for 15 minutes. After complete dehydration, the complex may also be restored to its initial state by adding moisture, like agar.

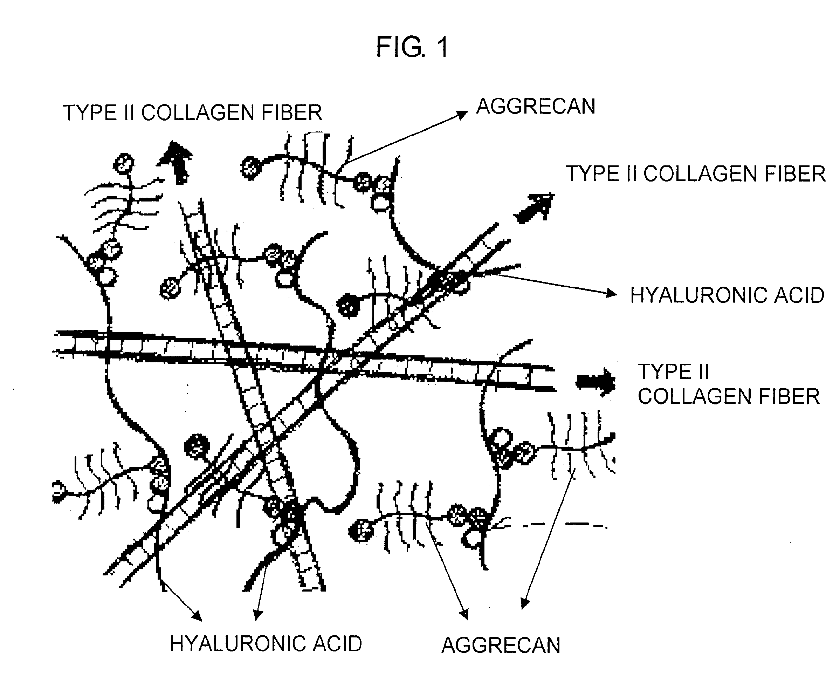

Self-organized glycosaminoglycan/proteoglycan/collagen complexes can be obtained by the methods of the present invention. Accordingly, the present invention also provides such self-organized glycosaminoglycan/proteoglycan/collagen complexes, and particularly provides self-organized glycosaminoglycan/proteoglycan/collagen cartilage-like complexes. The complexes of the present invention are produced using glycosaminoglycan, proteoglycan, and collagen by methods that utilize the self-organization techniques mentioned above. Thus, living cartilage tissues and matrices, extracts from vertebrate cartilage tissues, and complexes in those extracts are clearly excluded from the complexes of the present invention. The self-organized glycosaminoglycan/proteoglycan/collagen complexes of the present invention have structures resembling living tissues. In the present invention, the self-organized glycosaminoglycan/proteoglycan/collagen cartilage-like complexes are self-organized glycosaminoglycan/proteoglycan/collagen complexes that have a structure resembling living cartilage tissues. For example, in the Examples to be described, aggrecan (AG) and hyaluronic acid (HA) aggregated to form AG-HA aggregates, collagen molecules were regularly assembled around the aggregates, a mesh structure of fibrous type II collagen was formed, and then the complexes (hyaluronic acid/aggrecan/type II collagen complexes), which held a high density of hyaluronic acid-bound aggrecan in the structure, were formed. This bonding pattern mimics that of a living cartilage tissue (Fig. 1).

Moreover, the following points can also support that the complexes of the present invention mimic living tissues. The N-terminal globular G1 domain of an aggrecan has a lectin-like binding site which has high affinity to hyaluronic acid. It is known that, in a living cartilage tissue, aggrecan is bound densely to a non-branched single-chain hyaluronic acid having a molecular weight of several millions, through the binding site to form an aggregate. The length of hyaluronic acid in a living cartilage tissue varies. The present inventors have observed hyaluronic acids of about 500 nm to 10,000 nm (10 µm) long. Some examples by observation have reported the thickness of hyaluronic acid is about 20 to 50 nm. Collagen fibers formed by intermolecular bonding of collagen molecules have lengths of 0.1 to 500 µm (diameters of 2 to 50 nm) depending on the degree of polymerization, and form a dense mesh structure. As shown in the Examples, the complexes of the present invention are confirmed to have such structure.

The self-organized glycosaminoglycan/proteoglycan/collagen complexes of the present invention have physical properties which closely resemble those of living tissues. For example, living cartilage tissues have an elasticity of 0.1 to 0.5 GPa, and a friction coefficient of 0.01 to 0.001 (Robert P. Lanza, Robert Langer, and Joseph Vacanti, translation supervised by Noriya Oono and Masuo Aizawa, Principles of Regenerative Medicine, NTS Inc. pp. 203-206; Woo S. L.-Y., Mow V. C., and Lai W. M., Biomechanical properties of articular cartilage. "Handbook of Bioengineering" McGraw-Hill, New York, 1987). The hyaluronic acid/aggrecan/type II collagen complexes in the Examples are able to reproduce a maximum elasticity of 0.2 GPa and a friction coefficient of 0.05 to 0.005. The elasticity and friction coefficient can be measured using a commercially available hardness tester for soft solids and a friction and abrasion tester. For example, elasticity and friction can be measured using a hardness tester for soft solids and a friction and abrasion tester manufactured by Fuji Instruments Co., Ltd.

The above physical properties of the self-organized glycosaminoglycan/proteoglycan/collagen complexes of the present invention attribute to their structure which resembles that of living tissues at molecular level (nanocomposite). The important dynamic properties of living cartilage tissues, such as load-bearing property and compressive resistance are results of (1) a mesh structure of dense collagen fibers yielding tissue morphology and tensile property; (2) a high concentration of aggrecan which draws water into the tissue by an osmotic pressure to cause a swelling pressure in the collagen mesh structure; and (3) the function of hyaluronic acid (as an aggregate of hyaluronic acid bound with aggrecan) to hold aggrecan having such activity within cartilage tissues (collagen mesh tissues). Meanwhile, as described above, the aggregates of hyaluronic acid bound with aggrecan are formed in the glycosaminoglycan-proteoglycan aggregate preparation step in the methods of the present invention. Next, in the collagen molecule mixing step, collagen molecules are regularly assembled to form associated (intermolecularly bonded) collagen fibers to construct a mesh structure, within which a high density of these aggregates are held. The mesh structure is an important structure for providing comparable functions to those of living tissues, such as load resistance. The mesh structure can be confirmed with an electron microscope. Production of such structural bodies which mimic living cartilage tissues at nano level has become possible for the first time by the methods of the present invention. For example, it is known that a gel-like polyion complex (ionic conjugate) can be formed by mixing hyaluronic acid and a hydrochloric acid solution of collagen; however, the polyion complex does not have a cartilage tissue-like nano structure (Taguchi T., Ikoma T., and Tanaka J. An improved method to prepare hyaluronic acid and type II collagen composite matrices. J. Biomed. Mater. Res. 61(2):330-6, 2002).

As described above, the self-organized glycosaminoglycan/proteoglycan/collagen complexes and their production methods of the present invention are useful in producing materials for biological tissue regeneration. In particular, the self-organized glycosaminoglycan/proteoglycan/collagen cartilage-like complexes of the present invention are highly useful in the production of materials for treatment of cartilage defect and deformation, and materials for cartilage tissue regeneration. The complexes of the present invention are produced using cartilage matrix components so as to have approximately the same structure as that of a cartilage tissue; thus, they are very useful for the treatment of diseases that lead to cartilage defect or deformation (for example, osteoporosis, osteoarthritis, and arthritis such as rheumatic arthritis and rheumatoid-related diseases). Materials for the treatment of cartilage defect or deformation or materials for cartilage regeneration that use the self-organized glycosaminoglycan/proteoglycan/collagen cartilage-like complex of the present invention can be transplanted into living tissues with known methods. For example, a joint having defective or deformed cartilage may be incised and transplanted with the above material; alternatively, the above material may be injected with a syringe into a site having damaged cartilage. The dosage can be appropriately adjusted in accordance with the type and range of defective cartilage.

The complexes of the present invention are also useful as three-dimensional cell culture materials. As shown in the Examples, it was proven that three-dimensional cell culture was possible with the complex of the present invention. Accordingly, the complexes of the present invention can be materials for culturing cells, for which maintenance of three-dimensional structure is important, such as cells for transplantation. In particular, the complexes of the present invention are extremely useful for culturing cells, such as chondrocytes, which are easily dedifferentiated in a plate culture and have difficulties in long-term passage culture.

When chondrocytes are cultured using a complex of the present invention, the chondrocytes survive by using the complex of the present invention as a scaffold. Chondrocytes are transferred into the complexes of the present invention (hereinbelow, referred to as "chondrocyte-transferred complexes of the present invention") which can be smoothly engrafted; thus, the complexes of the present invention are extremely useful as materials for treatment of cartilage defect and deformation, and as materials for cartilage regeneration with an excellent biocompatibility. The *in vivo* self-organization of three-dimensionally cultured cartilage using a conventional collagen gel takes several weeks to several months, whereas in the present invention, it was confirmed that cells were transferred into the complex at the fourth week of incubation in *in vitro* assessment, and their engraftment was confirmed by autopsy at the sixth week in *in vivo* assessment. When a chondrocyte-transferred complex of the present invention is transplanted into a living body, in order to avoid rejection reaction, it is preferable to use a complex comprising chondrocytes derived from an animal of the same species as the living subject that will receive the transplantation. It is most preferable to collect cells from the living subject (patient) that will receive the transplantation, culture them with a complex of the present invention, and use the complex for transplantation. The use of autologous cells from patients can remarkably improve the safety of transplantation.

Moreover, the chondrocyte-transferred complexes of the present invention are also superior in terms of short preparation time and high biocompatibility. To provide a transplantable material through three-dimensional culturing of chondrocytes with a collagen, a large number of cells have to be cultured for a long incubation period conventionally. In contrast, the complexes of the present invention provide chondrocytes with a cellular environment close to the *in vivo* condition; therefore, the chondrocyte-transferred complexes can be prepared to be in a transplantable state by culturing a small number of cells with short incubation time. Specifically, conventional techniques require several weeks to prepare a required number of cells and another three to four weeks to do a three-dimensional culture with a collagen gel, whereas only 6 to 12 hours are required in the present invention for preparing the complex of the present invention through self-organization and only two to three hours are required for the operation of cell transfer.

When a chondrocyte-transferred complex of the present invention is used for treatment of cartilage defect or deformation, for example, a joint having defective or deformed cartilage may be incised and transplanted with the chondrocyte-transferred complex of the present invention, followed by suturing. The amount of chondrocyte-transferred complex of the present invention used for transplantation can be appropriately adjusted. For example, the volume may be adjusted according to the size of the defect or deformation.

All prior art documents cited in the present specification are incorporated herein by reference.

### Examples

### [Example 1] Production of hyaluronic acid/aggrecan/type II collagen complex

An attempt was made to produce a hyaluronic acid/aggrecan/type II collagen complex through self-organization techniques. For that purpose, the optimum conditions (concentration and pH) for hyaluronic acid, aggrecan, and collagen were examined.

### [1-1] Materials and Methods

Aggrecan (hereinbelow, may be referred to as "AG"; manufactured by Sigma Co., USA) was dissolved in distilled diluted water (hereinbelow, referred to as "DDW") as a solvent to prepare aggrecan solutions at various concentrations (the final concentration of aggrecan was 0.1, 0.25, 0.33, 0.5, or 1.0 mg/ml). Similarly, hyaluronic acid (hereinbelow, may be referred to as "HA"; manufactured by Chugai Pharmaceutical Co., Ltd., Japan; average molecular weight of 1,800,000) was dissolved in DDW to prepare hyaluronic acid solutions at various concentrations (final volume percent was 1, 2, 3, 4, or 5 volume percent). The AG solution and the HA solution were mixed to prepare AG and HA-dissolved solution (hereinbelow, referred to as "AG + HA solution"). Type II collagen molecule (manufactured by Collagen Research Association, Japan) was dissolved in DDW to prepare collagen solutions at various concentrations of 0.1, 0.25, 0.33, and 0.5 mg/ml. The AG + HA solution and the collagen solution were each adjusted to pH 4.0, 5.0, 6.0, 7.0, 7.5, 8.0, 8.5, 9.0, 9.5, 10.0, 10.5, or 11.0. Equal volumes of the AG + HA solution and collagen solution were mixed by simultaneous dripping at 37°C under these various concentrations and pH conditions, and complex formation was observed using a phase-contrast microscope over time. To reproduce an *in vivo* environment where a cartilage tissue is actually formed, they were mixed using an incubator without any factors such as light and ultraviolet rays at 37°C. The aggregates or complexes formed in each solution were dispersed in distilled water, and then were placed on a collodion film set up on a MicroGrid to prepare specimens for transmission electron microscopic observation.

### [1-2] Results

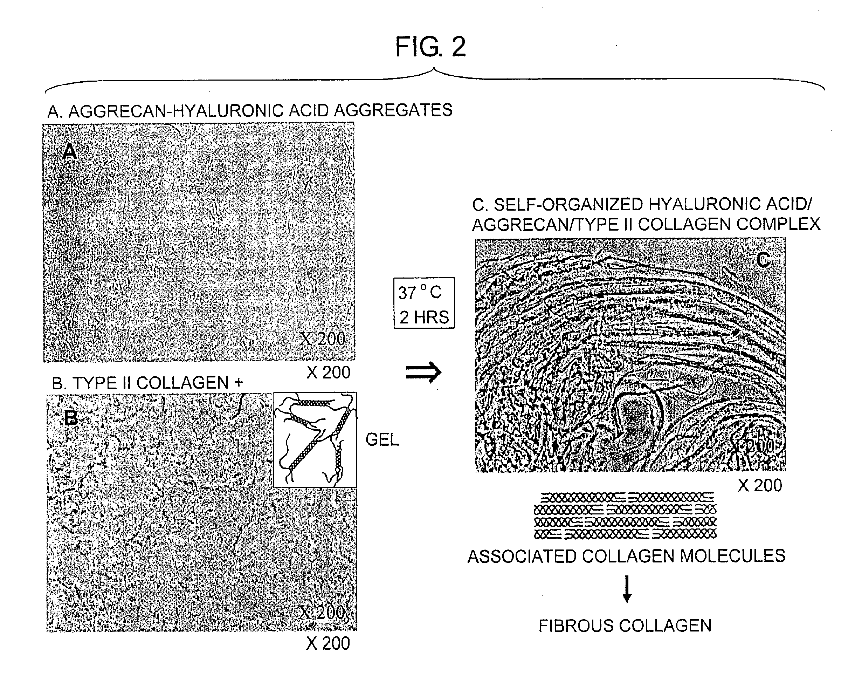

In the AG + HA solution, aggrecan-hyaluronic acid aggregates (hereinbelow, referred to as "AG-HA aggregates") were observed within a range of pH 6 to 9 under conditions of combinations between 0.25 and 0.5 mg/ml of AG and 1 and 5 volume percent of HA. These AG-HA aggregates, in particular, were remarkably observed within a range of pH 8 to 9, when mixing a 0.33 mg/ml AG solution with a 3% HA solution. Fig. 2A shows the result of mixing the AG solution (concentration of 0.33 mg/ml) at pH 9.0 and the 3% HA solution at pH 9.0. Table 1 shows the state of AG-HA aggregate formation in mixtures of the AG solution and HA solution at various pH. The trend indicated in Table 1 was observed with hyaluronic acid at concentrations of 1%, 2%, 3%, 4%, and 5%, and aggrecan at concentrations of 0.25, 0.33, and 0.5 ng/ml.

**[Table 1]**

| Hyaluronic acid | | | | | | | | | | | | | |
|---|---|---|---|---|---|---|---|---|---|---|---|---|---|
| | pH | 4 | 5 | 6 | 7 | 7.5 | 8 | 8.5 | 9 | 9.5 | 10 | 10.5 | 11 |
| | 4 | - | - | - | - | - | - | - | - | - | - | - | - |
| | 5 | - | - | - | - | - | - | - | - | - | - | - | - |
| | 6 | - | - | + | + | + | + | + | + | + | + | - | - |
| | 7 | - | - | + | + | + | + | + | + | + | + | - | - |
| | 7.5 | - | - | + | + | + | + | + | + | + | + | - | - |
| | 8 | - | - | + | + | + | ++ | ++ | ++ | + | + | - | - |
| Aggrecan | 8.5 | - | - | + | + | + | ++ | ++ | +++ | + | + | - | - |
| | 9 | - | - | + | + | + | ++ | ++ | ++++ | + | + | - | - |
| | 9.5 | - | - | + | + | + | + | + | +++ | + | + | - | - |
| | 10 | - | - | - | - | | + | + | + | - | - | - | - |
| | 10.5 | - | - | - | - | - | - | - | - | - | - | - | - |
| | 11 | - | - | - | - | - | - | - | - | - | - | - | - |

| | | | | | | | | | | | | | |
|---|---|---|---|---|---|---|---|---|---|---|---|---|---|
| - No formation + Slight formation ++ Moderate formation +++ Advanced formation ++++ Maximum formation within the test conditions | | | | | | | | | | | | | |

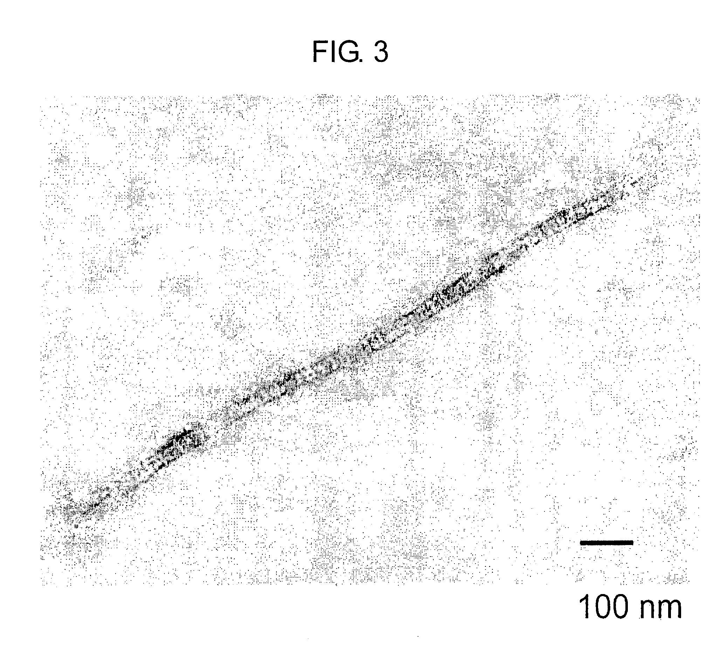

The transmission electron microscopic image showed a macroscopic fibrous structure, and an aggregate structure having an average of about 200 AGs bound to an HA molecule was observed microscopically (Fig. 3).

Similar results to the above were obtained with the use of hyaluronic acid having an average molecular weight of 900,000 (results not shown).

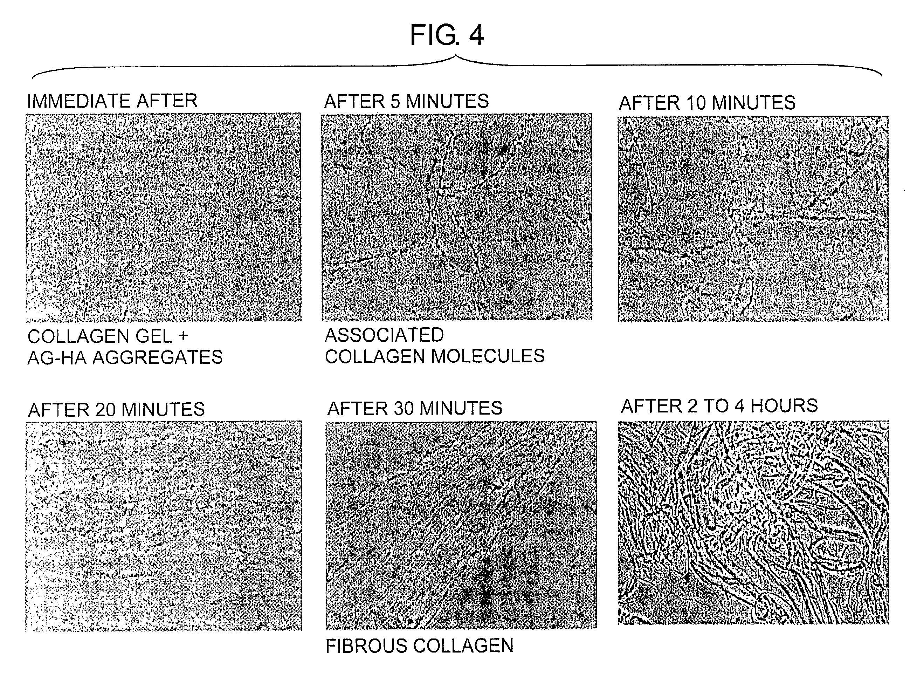

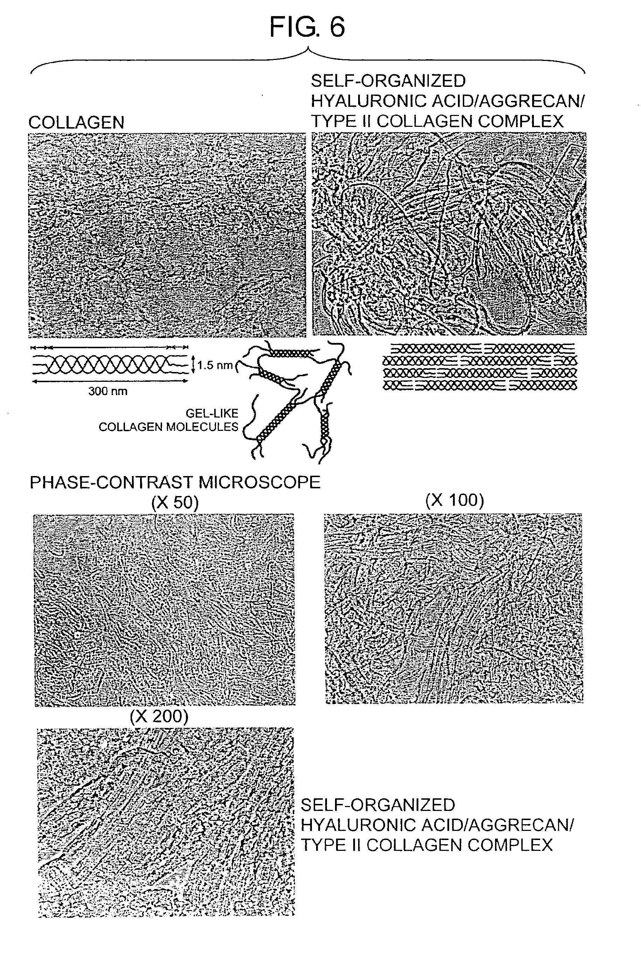

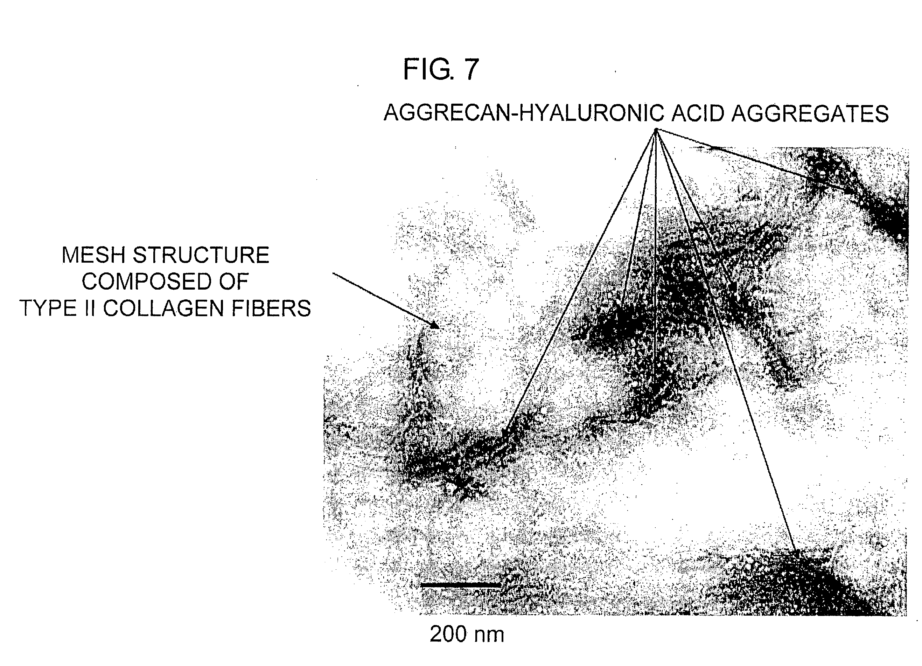

Immediately after the AG + HA solution (Fig. 2A) and the collagen solution (Fig. 2B) were mixed, it was observed that collagen molecules were regularly assembled and associated (intermolecularly bonded) around an AG-HA aggregate and began to form collagen fibers. After several minutes to several hours (two to three hours), extension and thickening of the fibrous collagen were observed (Fig. 2C and Fig. 4). Conventionally known collagen gels only have gel-like collagen branches, and do not have a solid structural organization. Unlike such collagen gels, the self-organized hyaluronic acid/aggrecan/type II collagen complex has a nanocomposite structure in which the thus-formed mesh structure composed of fibrous collagen holds a high density of hyaluronic acid-bound aggrecan. Such structure resembles a cartilage tissue (Fig. 6 and Fig. 7).

The self-organized hyaluronic acid/aggrecan/type II collagen complex organization comprising fibrous collagen having a length of 0.1 to 500 µm (diameter of 2 to 50 nm) was observed within a range of pH 6 to 10. Table 2 shows the relation between pH and complex formation using the mixture of AG-HA aggregates and type II collagen solution. The trend shown in Table 2 was observed with the type II collagen solution at concentrations of 0.25, 0.33, and 0.5 ng/ml.

**[Table 2]**

| | Type II collagen solution (the trend was found at concentrations of 0.25, 0.33, and 0.5 ng/ml) | | | | | | | | | | | | |
|---|---|---|---|---|---|---|---|---|---|---|---|---|---|
| | pH | 4 | 5 | 6 | 7 | 7.5 | 8 | 8.5 | 9 | 9.5 | 10 | 10.5 | 11 |
| Aggrecan + hyaluronic acid aggregate solution | 6 | - | - | - | - | - | + | + | + | + | + | - | - |
| | 7 | - | - | + | + | + | + | + | + | + | + | - | - |
| | 7.5 | - | - | + | + | + | + | + | + | + | + | - | - |
| | 8 | - | - | + | + | + | ++ | ++ | ++ | + | + | - | - |
| | 8.5 | - | - | + | + | + | ++ | ++ | +++ | + | + | - | - |
| | 9 | - | - | + | + | + | ++ | ++ | ++++ | + | + | - | - |
| | 9.5 | - | - | - | - | + | + | + | +++ | + | + | - | - |
| | 10 | - | - | - | - | - | - | + | + | - | - | - | - |
| | 10.5 | - | - | - | - | - | - | - | - | - | - | - | - |

| | | | | | | | | | | | | | |
|---|---|---|---|---|---|---|---|---|---|---|---|---|---|
| - No formation + Slight formation ++ Moderate formation +++ Advanced formation ++++ Maximum formation within the test conditions | | | | | | | | | | | | | |

Moreover, complex formation was remarkably observed when equal amounts (same volumes, 1:1) of the AG (0.33 mg/ml) + HA (3%) solution and the type II collagen molecule solution (0.25 to 0.5 mg/ml) were mixed (Fig. 2). Table 3 shows the association between complex formation and the blending ratio of the AG (0.33 mg/ml) + HA (3%) solution to the type II collagen molecule solution.

**[Table 3]**

| Hyaluronic acid/aggrecan/type II collagen complex-formation ability (± Minimum formation, + Slight formation, ++ Moderate formation, +++ Advanced formation, ++++ Maximum formation within the test conditions) | |
|---|---|
| Blending ratio (Aggrecan-hyaluronic acid aggregate : type II collagen) | |
| 5:1 | ± |
| 3:1 | ± |
| 2:1 | + |
| 1:1 | +++ |
| 1:2 | ++ |
| 1:3 | ++ |
| 1:5 | + |

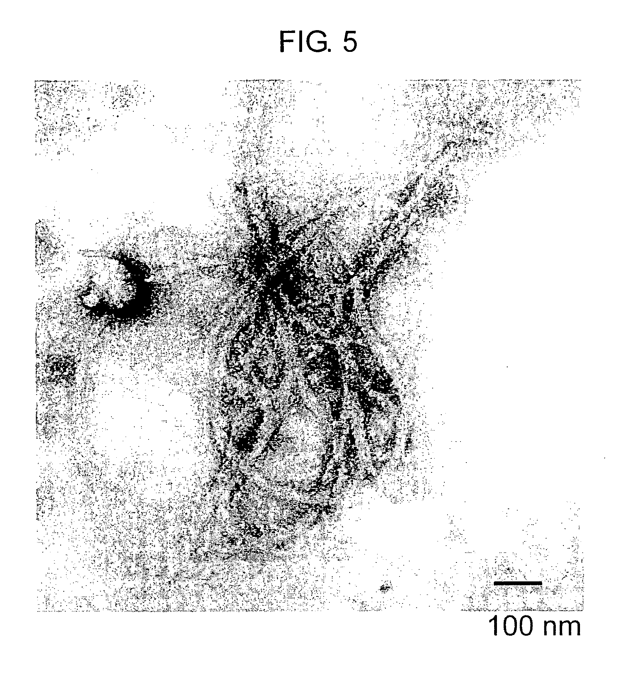

In the transmission electron microscopic images of this self-organized hyaluronic acid/aggrecan/type II collagen complex, it was confirmed that collagen molecules were regularly associated to form long fibers (Fig. 5 and Fig. 7).

From the above results, the optimum conditions for formation of the self-organized hyaluronic acid/aggrecan/type II collagen complex were found: mixing equal amounts (same volumes) of the AG (0.33 to 0.50 mg/ml) + HA (3 volume percent) solution and the type II collagen molecule solution (0.25 to 0.5 mg/ml) at 37°C and pH 6 to 9, particularly at pH 9. Complex formation was observed immediately after these solutions were mixed, and two to three hours were required for sufficient complex formation.

### [1-3] Assessment of complexes' physical properties

Living cartilage tissues are thought to have an elasticity of 0.1 to 0.5 GPa and a friction coefficient of 0.01 to 0.001. The elasticity and friction coefficient of the self-organized hyaluronic acid/aggrecan/type II collagen complexes produced by the above Example were measured. The elasticity and friction coefficient were measured using a commercially available hardness tester for soft solids and a friction and abrasion tester (both manufactured by Fuji Instruments Co., Ltd.). The above self-organized hyaluronic acid/aggrecan/type II collagen complexes had a maximum elasticity of 0.2 GPa and a friction coefficient of 0.05 to 0.005. The self-organized hyaluronic acid/aggrecan/type II collagen complexes were confirmed to have similar physical properties to living cartilage tissues.

### [Example 2] Experiment of cell transfer into self-organized complexes

The complex of the present invention desirably has high bioaffinity in view of its application to cartilage tissue regeneration. Specifically, it is important that an individual's chondrocytes can be engrafted when administered to a joint. Therefore, the affinity between cells and the complex of the present invention was examined by culturing chondrocytes using the complex of the present invention.

### [2-1] Production of complexes comprising cultured chondrocytes

If blood serum is added to a cell culture solution for culturing chondrocytes, various factors in the blood serum would affect the formation and maintenance of the complexes and the engraftment of cells in the complexes, which may make it difficult for appropriate assessment. To avoid such effects, neutridomas were added to a serum-free medium DMEM at a final concentration of 10% to prepare a 10% neutridoma-containing DMEM solution. Rat and human chondrocytes were cultured using this solution as a medium for culturing rat or human chondrocytes.

The self-organized glycosaminoglycan/proteoglycan/collagen complexes produced in the above method described in Example 1 were washed three times with the 10% neutridoma-containing DMEM solution, and then the culture solution was replaced with the 10% neutridoma-containing DMEM solution. The solution was warmed in an incubator set at 37°C. The cultured chondrocytes suspended in the above medium were added at a concentration of 5 x 10⁴ cells/ml. The solution was gently shaken and then centrifuged at 3,000 rpm for three minutes at a room temperature. After the centrifugation, the culture supernatant was discarded, and a fresh 10% neutridoma-containing DMEM solution was added. The resultant solution was further centrifuged at 3,000 rpm for three minutes at room temperature. The culture supernatant was again discarded, and the solution was replaced with another fresh 10% neutridoma-containing DMEM solution. The resultant solution was incubated in a 5% CO₂ incubator at 37°C.

### [2-2] Confirmation of chondrocytes in self-organized complexes by optical microscopic observation

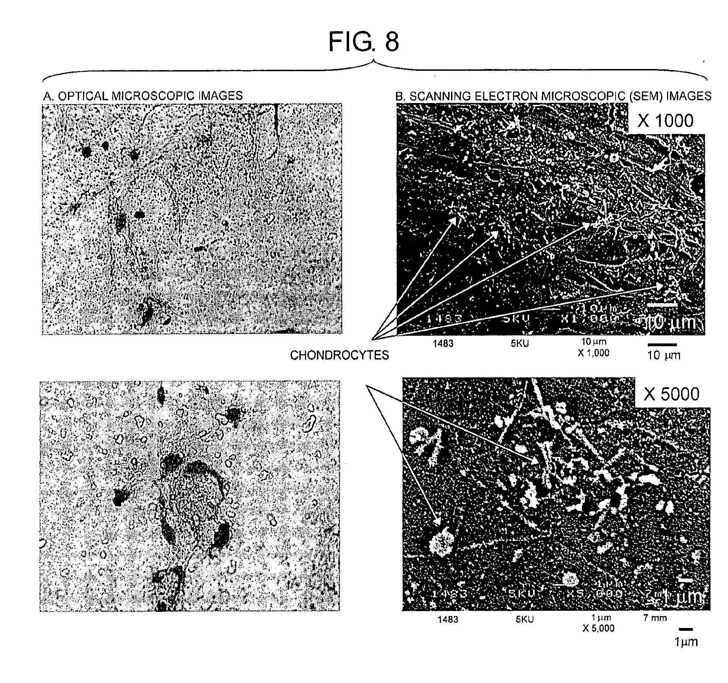

The chondrocyte-transferred self-organized complexes prepared in [2-1] above were incubated at 5% CO₂ and 37°C. Time-course observation was performed with a phase-contrast microscope during incubation (Fig. 8A). In the first week of incubation, the self-organized complexes were taken out and fixed with 4% paraformaldehyde, followed by embedding in paraffin-paraffin. The specimen was sliced and subjected to hematoxylin-eosin staining and safranin-O staining, followed by optical microscopic observation.

As a result, it was observed that the self-organized complexes were held at a high density, and chondrocytes were evenly present using the complex-forming fibers as a scaffold. It was confirmed that the chondrocytes extended their dendrites to be engrafted in the complexes, and were alive in the tissue.

### [2-3] Electron microscopic observation of chondrocyte-transferred self-organized complexes

The chondrocyte-transferred self-organized complexes produced according to the method of [2-1] above were incubated in a 5% CO₂ incubator at 37°C for four weeks. Then, the complexes were taken out to produce the specimens for transmission and scanning electron microscopic observation. Since the transmission electron microscope (TEM) captures only cross-sectional or fragmentary images of collagen fibers in the complexes, the specimens were subjected to block staining with tannic acid.

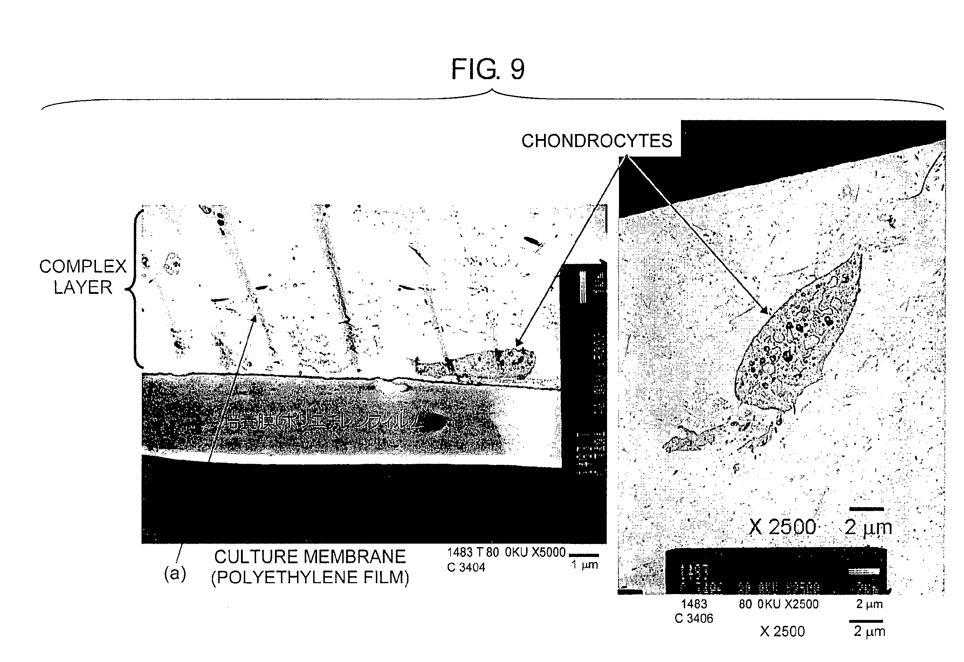

A highly dense structure of the self-organized complexes and chondrocytes present on or inside the complexes were observed in the scanning electron microscopic images. The images show that the chondrocytes extended their dendrites to be engrafted in the complexes. The transmission electron microscopic images show the intracellular organization of chondrocytes within the self-organized complexes, suggesting chondrocyte survival in the tissue (Fig. 8B and Fig. 9: transmission and scanning electron microscopic images).

It was confirmed from the above that, because of the three-dimensional mesh structure, the self-organized cartilage-like complexes of the present invention have a function of holding/containing liquid components such as an optimum culture solution for cell culture, and as a scaffold for the growth of chondrocytes, can reproduce a three-dimensional environment suitable for long-term survival of chondrocytes.

### [Example 3] Transplantation of self-organized complexes into knee joints of experimental animals

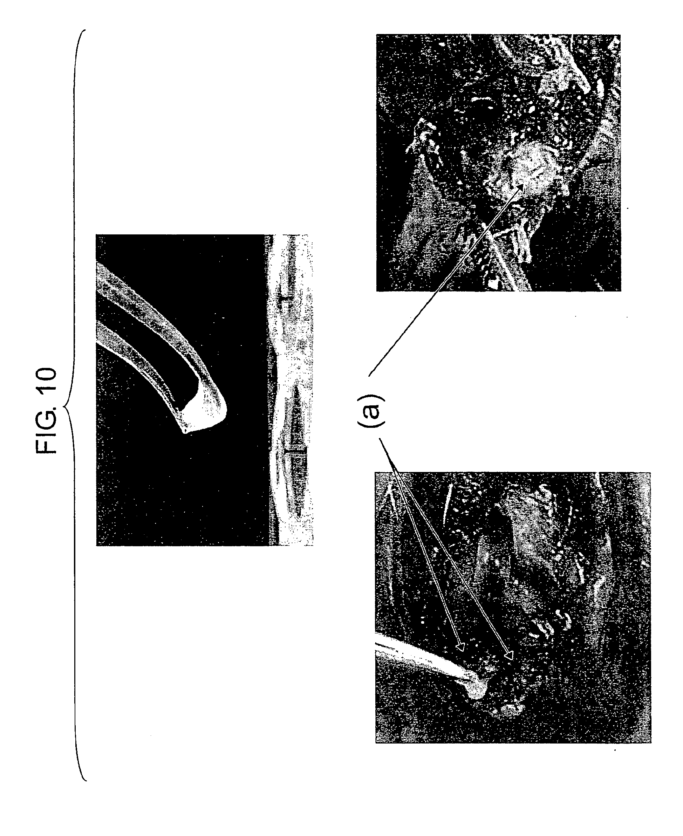

The self-organized complexes were produced using rat-derived type II collagen and aggrecan according to the method described in Example 1 above. Four 12-week-old male SD rats were anaesthetized with ether and then sterilized. Both of their knee joints were incised by aseptic techniques. The surface of articular cartilage of the medial and lateral (internal and external) malleoluses was pierced with an 18-gauge injection needle, to produce two articular cartilage defects per knee joint. The self-organized complexes were transplanted into one of these two articular cartilage defects (Fig. 10). The joint tissues were sutured, and the rats were grown for six weeks. In the sixth week, the mice were euthanized. The knee articular cartilage tissues were collected from them and fixed with 4% paraformaldehyde, followed by embedding in paraffin-paraffin. The specimen was sliced and subjected to hematoxylin-eosin staining and safranin-O staining, followed by optical microscopic observation.

It was observed that the self-organized complexes in the sixth week still maintained the same structure when they were prepared. This result shows that these quickly-producible self-organized complexes are useful as biomaterials for cartilage regeneration medicine against articular cartilage defects.

### Industrial Applicability

The present invention has provided self-organized glycosaminoglycan/proteoglycan/collagen complexes and their production methods. The self-organized glycosaminoglycan/proteoglycan/collagen complexes of the present invention are produced through self-organization techniques; thus, the complexes do not contain chemical substances such as crosslinking agents. Moreover, the structure of the collagen polysaccharide complexes of the present invention resembles that of living tissues at nano level. Accordingly, the self-organized glycosaminoglycan/proteoglycan/collagen complexes of the present invention resemble living tissues also in terms of structure-based physical properties. The self-organized glycosaminoglycan/proteoglycan/collagen complexes of the present invention have extremely high safety and functionality as biomaterials for tissue regeneration.

## Claims

1. A method for producing a self-organized glycosaminoglycan/proteoglycan/collagen complex, comprising steps (a) and (b) below:
(a) a step of preparing a glycosaminoglycan-proteoglycan aggregate by mixing glycosaminoglycan with proteoglycan; and
(b) a step of mixing collagen with said glycosaminoglycan-proteoglycan aggregate.

2. A method for producing a cartilage-like complex, comprising steps (a) and (b) below:
(a) a step of preparing a glycosaminoglycan-proteoglycan aggregate by mixing glycosaminoglycan with proteoglycan; and
(b) a step of mixing collagen with said glycosaminoglycan-proteoglycan aggregate to produce a cartilage-like self-organized glycosaminoglycan/proteoglycan/collagen complex.

3. A method for producing a cartilage matrix-like complex, comprising steps (a) and (b) below:
(a) a step of preparing a glycosaminoglycan-proteoglycan aggregate by mixing glycosaminoglycan with proteoglycan; and
(b) a step of mixing collagen with said glycosaminoglycan-proteoglycan aggregate to produce a cartilage matrix-like self-organized glycosaminoglycan/proteoglycan/collagen complex.

4. The production method of any one of claims 1 to 3, wherein the glycosaminoglycan is hyaluronic acid.

5. The production method of any one of claims 1 to 4, wherein the proteoglycan is aggrecan.

6. The production method of any one of claims 1 to 5, wherein the collagen is type II collagen.

7. The method of any one of claims 4 to 6, wherein the hyaluronic acid is a hyaluronic acid solution at pH 5 to pH 10 in step (a).

8. The method of any one of claims 5 to 7, wherein the aggrecan is an aggrecan solution at pH 5 to pH 10 in step (a).

9. The method of claim 1, wherein the collagen is a collagen solution at pH 5 to pH 10 in step (b).

10. The method of any one of claims 4 to 6, wherein the hyaluronic acid is a hyaluronic acid solution at a concentration of 20 volume percent or less in step (a).

11. The method of any one of claims 5 to 7, wherein the aggrecan is an aggrecan solution at a concentration of 0.1 to 1.0 mg/ml in step (a).

12. The method of claim 1, wherein the collagen is a collagen solution at a concentration of 0.1 to 5.0 mg/ml in step (b).

13. A self-organized glycosaminoglycan/proteoglycan/collagen complex produced by the method of any one of claims 1 and 4 to 12.

14. A complex comprising hyaluronic acid, aggrecan, and type II collagen, which has a mesh structure formed by linkage between a type II collagen fiber and an aggregate of hyaluronic acid-bound aggrecan.

15. A cartilage-like complex produced by the method of any one of claims 2 and 4 to 12.

16. A cartilage matrix-like complex produced by the method of any one of claims 3 to 12.

17. A material for cartilage tissue regeneration or treatment of cartilage damage or cartilage degeneration, comprising the complex of any one of claims 13 to 16.

18. A method for producing a chondrocyte-comprising material for treatment of cartilage damage or cartilage degeneration, comprising steps (a) to (c) below:
(a) a step of preparing a glycosaminoglycan-proteoglycan aggregate by mixing glycosaminoglycan with proteoglycan;
(b) a step of mixing collagen with said glycosaminoglycan-proteoglycan aggregate to produce a self-organized glycosaminoglycan/proteoglycan/collagen complex; and
(c) a step of culturing a chondrocyte using said self-organized glycosaminoglycan/proteoglycan/collagen complex.

19. The production method of claim 18, wherein the chondrocyte is derived from a patient who receives treatment of cartilage damage or cartilage degeneration.

20. A chondrocyte-comprising material for treatment of cartilage damage or cartilage degeneration produced by the method of claim 18 or 19.

21. A three-dimensional cell culture method, comprising a step of preparing a complex according to the method of claim 1, and a step of culturing a cell using said complex.

22. The three-dimensional culture method of claim 21, wherein the cell is a chondrocyte.

23. A therapeutic method for a disease involving cartilage damage or cartilage degeneration, comprising a step of administering the material of claim 17 or 20 to a joint having cartilage damage or cartilage degeneration.

24. Use of the complex of claim 13 or 14 for the production of a material for treatment of cartilage damage or cartilage degeneration.

25. A three-dimensional cell culture material, comprising the complex of claim 13 or 14.
